# EUROPÄISCHE PATENTSCHRIFT

(11) **EP 2 727 551 B1**
(45) Veröffentlichungstag und Bekanntmachung des Hinweises auf die Patenterteilung: **20.05.2015**
(21) Anmeldenummer: 13190925.1
(22) Anmeldetag: 30.10.2013
(51) Int. Cl.: A61B 19/02

(54) **Behandlungseinheit für medizinische Arbeitsplätze**
Treatment unit for medical workstations
Unité de traitement pour postes de travail médicaux

(30) Priorität: 30.10.2012 DE 102012021184
(43) Veröffentlichungstag der Anmeldung: 07.05.2014
(73) Patentinhaber: Happersberger Otopront GmbH, 65327 Hohenstein (DE)
(72) Erfinder: Happersberger, Rainer, 65329 Hohenstein (DE)
(74) Vertreter: Keil & Schaafhausen Patent- und Rechtsanwälte

(56) Entgegenhaltungen:
- DE-A1- 3 202 304
- DE-A1-102009 002 645
- DE-U1- 9 114 206
- FR-A7- 2 268 437
- JP-A- H03 170 107

## Beschreibung

Die Erfindung betrifft eine Behandlungseinheit, insbesondere einen Instrumentenschrank für medizinische Arbeitsplätze mit mindestens einer herausziehbaren Schublade und/oder einer Instrumentenablageplatte, die in Ausnehmungen in der Behandlungseinheit auf seitlichen Schienen gelagert sind.

Bei Behandlungseinheiten im medizinischen Bereich werden Ablageflächen für eine übersichtliche und stets griffbereite Unterbringung von Instrumenten und Zubehör benötigt. Die obere Ablagefläche einer Behandlungseinheit wird dabei üblicherweise durch eine hochklappbare transparente Abdeckung vor Umgebungseinflüssen geschützt. Die in den Schubladen eines Instrumentenschrankes angeordneten Instrumente und Zubehör unterliegen ebenfalls den Umgebungseinflüssen, wozu insbesondere mit Krankheitskeimen von in dem Behandlungsraum sich aufhaltenden Personen zu rechnen sind. Es sind daher Bestrebungen im Gange, zu fordern, dass Schubladen bei derartigen Behandlungseinheiten ständig abzudecken sind. Aber auch die bei der oberen Ablagefläche bereits bekannte Abdeckung nützt wenig, wenn sie wegen der umständlichen Handhabung nicht verwendet wird. So bleibt die transparente Abdeckung häufig geöffnet und wird, wenn überhaupt, nur bei Beendigung der Behandlung geschlosses. Dokument FR-A-2268437 offenbart ein Behandlungseinheit gemäß dem Oberbegriff des Anspruchs 1.

Aufgabe der vorliegenden Erfindung ist es daher, eine einfach und schnell verschließbare Abdeckung sowohl für die obere Ablagefläche als auch für die Schubladen von Behandlungseinheiten bzw. einem dazugehörigen Instrumentenschrank eines medizinischen Arbeitsplatzes zur Verfügung zu stellen.

Diese Aufgabe wird erfindungsgemäß dadurch gelöst, dass die Schublade und/oder die Instrumentenablageplatte einer Behandlungseinheit mit einem Schutzdeckel über wenigstens eine elastische Gliederkette bewegbar verbunden ist, deren eines Ende am hinteren Ende der Schublade und/oder der Instrumentenablageplatte und deren anderes Ende am hinteren Ende des Schutzdeckels befestigt ist, dass an den Seitenwänden der Ausnehmung in der Behandlungseinheit Führungsplatten angeordnet sind, in denen wenigstens eine annähernd U-förmige Führungsnut ausgebildet ist, in der die Gliederkette geführt ist, dass an den Seiten des Schutzdeckels Stützrollen angeordnet sind, die in die Führungsnut(en) eingreifen, dass die Führungsnut abgewinkelte Abschnitte aufweist und dass bei einer Bewegung der Stützrollen entlang der abgewinkelten Abschnitte der Führungsnut der Schutzdeckel je nach Bewegungsrichtung der Gliederkette angehoben oder abgesenkt wird.

Damit wird eine sichere Abdeckung der medizinischen Instrumente und des Zubehörs in Behandlungsräumen gewährleistet, die so einfach zu handhaben ist, dass sie ohne Beeinträchtigung der Behandlung einfach benutzt werden kann und damit zur Sicherheit in einem Behandlungsraum beiträgt.

Um einen einfach zu handhabenden Schnellverschluss für die Schublade einer Behandlungseinheit, insbesondere eines Instrumentenschrankes zur Verfügung zu stellen, ist gemäß einer Ausführungsform der Erfindung vorgesehen, dass in der die herausziehbare Schublade aufnehmenden Ausnehmung in der Behandlungseinheit zwei parallel zueinander verlaufende abgewinkelte Führungsnut-Abschnitte in der seitlichen Führungsplatte ausgebildet sind, in die die an dem Schutzdeckel angeordneten Stützrollen eingreifen. Damit wird erreicht, dass beim Herausziehen der Schublade aus der Behandlungseinheit bzw. dem Instrumentenschrank der Schutzdeckel über die Stützrollen gleichmäßig angehoben und gleichzeitig in gegenläufiger Richtung zur Schublade zurückgezogen und die Schublade frei zugänglich wird. Wird die Schublade wieder in die Schubladenaufnahme hineingeschoben, wird der Schutzdeckel mittels der Gliederkette in umgekehrter Richtung bewegt und auf die Schublade abgesenkt. In dieser Endlage ist die Schublade staubdicht verschlossen.

Um auch für die Instrumentenablagefläche der Behandlungseinheit, bei der es bereits bekannt ist, eine transparente Kunststoffabdeckung vorzusehen, die nach oben verschwenkbar ist und die als herausziehbare Ablageplatte ausgebildet ist, ist gemäß einer weiteren Ausführungsform der Erfindung vorgesehen, einen entsprechenden Schnellverschluss für die Abdeckung zur Verfügung zu stellen. Erfindungsgemäß ist vorgesehen, dass die als Instrumentenablage dienende herausziehbare Ablageplatte mit einem aufschwenkbaren hinteren Schutzdeckel verbunden ist, der mit einem vorderen Schutzdeckel die Abdeckung der Ablageplatte bildet, dass die Ablageplatte mit dem vorderen Schutzdeckel über wenigstens eine elastische Gliederkette bewegbar verbunden ist, deren eines Ende am hinteren Ende der Ablageplatte und deren anderes Ende am hinteren Ende des vorderen Schutzdeckels befestigt ist, dass an den Seiten des vorderen Schutzdeckels Stützrollen angeordnet sind, die in Führungsnuten eingreifen, die in den Seitenwänden der Ausnehmung in der Behandlungseinheit und in Seitenwandabschnitten des hinteren Schutzdeckels ausgebildet sind, dass die Führungsnut abgewinkelte Abschnitte aufweist und dass bei einer Bewegung der Stützrollen entlang der abgewinkelten Abschnitte der Führungsnut der vordere Schutzdeckel abgesenkt und unter den hinteren Schutzdeckel gezogen wird. Damit lässt sich bei einem Vorziehen der Ablageplatte die Abdeckung im Bereich des vorderen Schutzdeckels öffnen und bei einem Zurückschieben der Ablageplatte auch wieder luftdicht verschließen.

Dieser Verschließvorgang wird dadurch erleichtert, dass der vordere Schutzdeckel seitlich angeordnete Kurvenscheiben aufweist, die mit an der Führungsplatte angeordneten Andrückrollen in Eingriff treten. Damit wird der vordere Schutzdeckel in der Endphase seiner Schließbewegung durch die Bewegung der Kurvenscheibe entlang der Andrückrolle zwangsweise nach oben in seine endgültige Schließstellung gedrückt.

Gemäß einer weiteren Ausgestaltung der Erfindung ist vorgesehen, dass der hintere Schutzdeckel bei zurückgezogenem vorderem Schutzdeckel mit dem darunter positionierten vorderen Schutzdeckel aufschwenkbar ist. Dadurch wird die Reinigung der oberen Instrumentenablagefläche der Behandlungseinheit wesentlich erleichtert.

Es hat sich als zweckmäßig erwiesen, dass die hinteren Stützrollen an den Seiten des mit der Gliederkette verbundenen verschiebbaren Schutzdeckels eine Kettenaufnahme aufweisen. Damit wird eine sichere Führung der Kette in der seitlichen Führungsnut gewährleistet.

Es hat sich als vorteilhaft erwiesen, dass die Behandlungseinheit erfindungsgemäß einen Riegelmechanismus aufweist, der das Einschieben der Schublade bei geöffnetem hinterem Schutzdeckel blockiert. Damit wird eine Beschädigung der elastischen Gliederkette verhindert, die sich bei geöffnetem Schutzdeckel nicht in den hinteren Bereich der Führungsnut bewegen könnte, so dass die Gliederkette beschädigt würde. Eine solche Bewegung ist nur dann möglich, wenn der hintere Schutzdeckel abgesenkt ist. Jetzt kann der vordere Schutzdeckel unter dem hinteren Schutzdeckel hervor nach vorne in seine Abdeckposition gebracht worden ist.

Um einen sicheren Bewegungslauf der elastischen Gliederkette zu gewährleisten, ist diese als halbsteife Kette ausgebildet, die nur in einer Richtung verformbar ist. Damit der Schutzdeckel gleichmäßig geführt wird, greifen an beiden Seiten des Schutzdeckels Gliederketten an, die in jeder der Führungsnuten der gegenüberliegenden Führungsplatten eingreifen.

Gemäß einer weiteren vorteilhaften Ausgestaltung der Erfindung ist vorgesehen, dass die Schutzdeckel zumindest im vorderen Teil aus einem transparenten Kunststoff bestehen. Damit kann man auch bei geschlossener Abdeckung den Inhalt der Ablagefläche bzw. der Schublade sehen.

Die feste Verbindung der Gliederkette mit der Schublade erlaubt nur ein begrenztes Herausziehen der Schublade bis zu einem ersten Schubladenanschlag. Damit die Schublade über diesen Anschlag weiter herausgezogen werden kann, ist gemäß einer weiteren Ausgestaltung der Erfindung zwischen der Schublade und/oder der Instrumentenablageplatte und der Gliederkette eine lösbare Verbindung vorgesehen.

In Weiterbildung dieses Erfindungsgedankens ist vorgesehen, dass an dem der Schublade und/oder der Instrumentenablageplatte zugeordneten Ende der Gliederkette eine Mitnahmestange angeordnet, die mit einem an der Schublade bzw. der Instrumentenablageplatte angebrachten Magnet eine lösbare Verbindung bildet.

Zwischen dem der Schublade und/oder der Instrumentenablageplatte zugewandten Ende der Gliederkette kann erfindungsgemäß auch eine Schnappverbindung vorgesehen sein. Damit lässt sich die Schublade vollständig bis zu einem zweiten Schubladenanschlag herausziehen.

Die Haltekraft der lösbaren Verbindung muss dabei so beschaffen sein, dass sie sich bei einem (normalen) Herausziehen der Schublade solange festhält, bis die Mitnahmestange den Endanschlag erreicht hat. Hat die Mitnahmestange den Endanschlag erreicht, sollte die zusätzlich aufzuwendende Kraft, um die Verbindung zu lösen, nicht zu groß und störend sein.

Weitere Merkmale, Vorteile und Anwendungsmöglichkeiten der Erfindung ergeben sich aus der nachfolgenden Beschreibung zweier bevorzugter Ausführungsbeispiele, die in den Zeichnungen dargestellt sind.

Es zeigen:
- Fig. 1: in einer schematischen Seitenansicht eine erste Ausführungsform der Erfindung bestehend aus einer Schublade einer Behandlungseinheit mit einer aus einem Schutzdeckel bestehenden Abdeckung (geschlossen),
- Fig. 2: eine Fig. 1 entsprechende Darstellung der Schublade mit einem angehobenen Schutzdeckel,
- Fig. 3: eine Fig. 1 entsprechende Darstellung der Schublade mit einem geöffneten Schutzdeckel,
- Fig. 4: in einer Fig. 1 entsprechenden Darstellung eine zweite Ausführungsform der Erfindung bestehend aus einer Instrumentenablageplatte einer Behandlungseinheit mit einer aus zwei Schutzdeckeln bestehenden geschlossenen Abdeckung,
- Fig. 5: eine Fig. 4 entsprechende Darstellung der Ablageplatte mit einem angehobenen und unter den hinteren Schutzdeckel gezogenen vorderen Schutzdeckel,
- Fig. 6: eine Fig. 4 entsprechende Darstellung der Ablageplatte mit einer aufgeklappten Abdeckung,
- Fig. 7: in einer Fig. 1 entsprechenden Darstellung eine weitere Ausführungsform der Erfindung mit einer magnetischen Kettentrennung (geschlossener Schutzdeckel und geschlossene Schublade),
- Fig. 8: eine Fig. 1 entsprechende Darstellung der Erfindung mit einer magnetischen Kettentrennung (geöffneter Schutzdeckel und bis zum ersten Schubladenanschlag geöffnete Schublade),
- Fig. 9: eine Fig. 1 entsprechende Darstellung der Erfindung mit einer magnetischen Kettentrennung (geöffneter Schutzdeckel und bis zum zweiten Schubladenanschlag geöffnete Schublade),

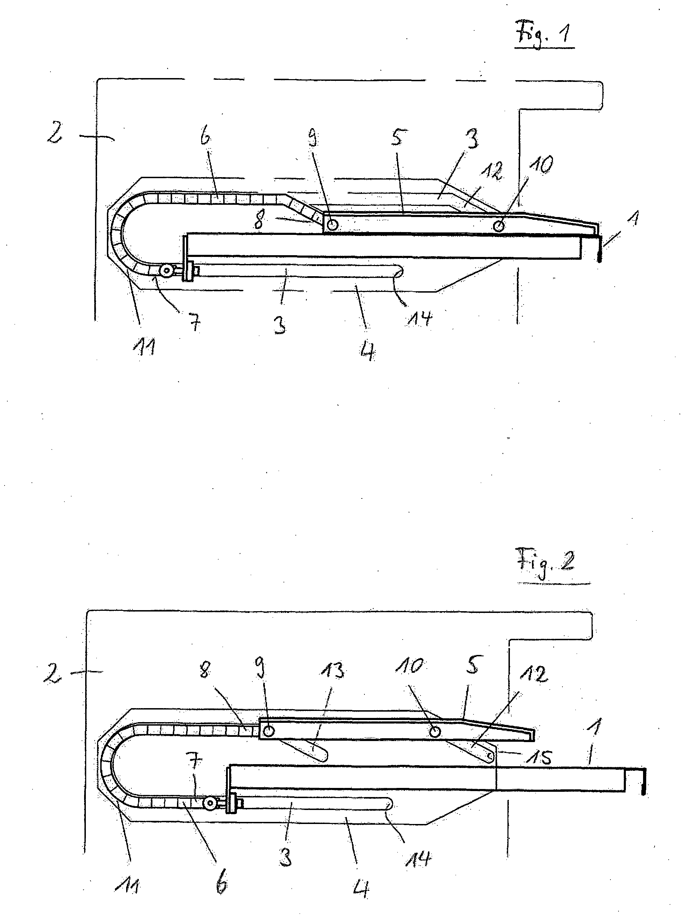

In der Fig. 1 ist eine Schublade 1 dargestellt, die in einer nur angedeuteten Ausnehmung 2 in einer nicht dargestellten Behandlungseinheit, insbesondere eines Instrumentenschrankes für medizinische Arbeitsplätze angeordnet ist. Die Schublade 1 ist ausziehbar und weist vorteilhafterweise eine Teleskopschienenführung auf. Ein Schutzdeckel 5 wird in einer Führungsnut 3 geführt, die in einer Führungsplatte 4 ausgebildet ist, die an den Seitenwänden der als Schubladenaufnahme dienenden Ausnehmung 2 in der Behandlungseinheit bzw. dem Instrumentenschrank angeordnet sind.

Die Schublade 1 ist mit einem Schutzdeckel 5 über eine elastische Gliederkette 6 bewegbar verbunden. Die Gliederkette 6 ist in der Führungsnut 3 geführt und mit ihrem einen Ende 7 mit der Schublade 1 und mit dem anderen Ende 8 mit dem Schutzdeckel 5 verbunden. Die Kettenaufnahme ist Bestandteil einer an dem Schutzdeckel 5 angebrachten hinteren Stützrolle 9, die zusammen mit einer im vorderen Bereich des Schutzdeckels 5 angeordneten Stützrolle 10 die Bewegung des Schutzdeckels 5 steuern.

Die Führungsnut 3 weist eine im Wesentlichen U-förmige Form auf mit einer Umlenkung 11 im hinteren Bereich der Ausnehmung 2 der Schubladenaufnahme. Im Bereich des Schutzdeckels 5 endet die Führungsnut 3 in einer Abwinklung 12. Im Abstand der beiden Stützrollen 9 und 10 ist ein parallel zur Abwinklung 12 verlaufender Führungsnut-Abschnitt 13 in der seitlichen Führungsplatte 4 ausgebildet. In diese beiden abgewinkelten Führungsnut-Abschnitte 12 und 13 greifen die Stützrollen 9 und 10 ein und sorgen dafür, dass bei einem Herausziehen der Schublade 1 der Schutzdeckel 5 über die Stützrollen 9 und 10 angehoben und in gegenläufiger Richtung in die Ausnehmung 2 zurückgezogen wird. Bei der gegenläufigen Bewegung, bei der die Schublade 1 wieder in die Ausnehmung zurückgeschoben wird, kehrt sich der Vorgang um und der Schutzdeckel 5 wird mittels der in den parallel verlaufenden abgewinkelten Führungsnut-Abschnitten 12, 13 geführten Stützrollen 9, 10 wieder bis zur völligen Abdeckung über die Schublade 1 gebracht.

Die Führung der elastischen Gliederkette 6 in der Führungsnut 3 wird dadurch stabilisiert, dass die Gliederkette als halbsteife Kette ausgebildet ist, die nur in einer Richtung verformbar ist.

Eine gleichförmige Bewegung der Schublade und des Deckels wird dadurch erreicht, dass in jeder Seitenwand der Ausnehmung 2 eine Führungsplatte 4 mit entsprechenden Führungsnuten 3 ausgebildet sind, in denen jeweils eine elastische Gliederkette 6 in gleicher Weise geführt werden.

Um leicht erkennen zu können, was sich in der Schublade 1 befindet, ist diese nach vorne hin offen ausgebildet. Außerdem besteht der Schutzdeckel 5 aus einem transparenten Kunststoff.

Die Funktionsweise der beschriebenen Schutzdeckel-Verschlusseinrichtung ergibt sich aus den Fig. 1 bis 3. In Fig. 1 ist die Schublade 1 in die Ausnehmung 2 eingeschoben und der Schutzdeckel 5 liegt auf der Schublade 1 auf und verschließt diese. Damit schützt der aus einem transparenten Kunststoff bestehende Schutzdeckel 5 die sich in der Schublade 1 befindlichen Instrumente und dgl. vor Umgebungseinflüssen. Wird nun, wie in Fig. 2 dargestellt, die Schublade 1 aus der Ausnehmung 2 herausgezogen, werden die Stützrollen 9 und 10 entlang der abgewinkelten Führungsnut-Abschnitte 12 und 13 geführt und heben dabei den Schutzdeckel 5 gleichförmig an. Der Schutzdeckel 5 wird dann entlang der waagerecht verlaufenden Führungsnut 3 weiter in die Ausnehmung hineingezogen und gibt dadurch die Schublade 1 frei.

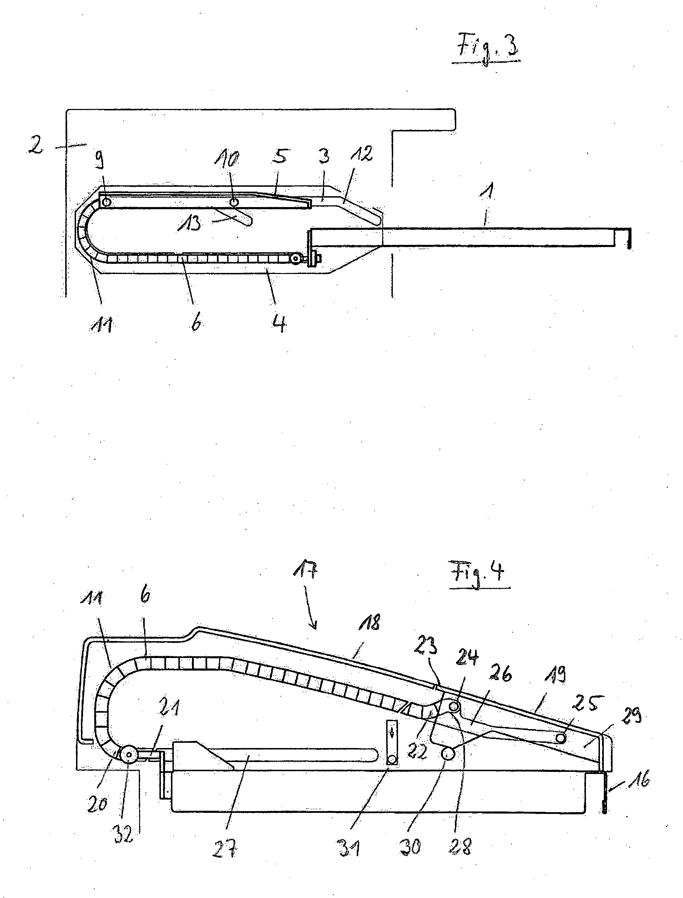

Fig. 3 zeigt die Endlage bei vollständig herausgezogener Schublade 1 und vollständig in die Ausnehmung 2 zurückgezogenem Schutzdeckel 5. Der Bewegungsweg wird dabei durch den Endanschlag 14 begrenzt.

Wird die Schublade 1 wieder in die Ausnehmung 2 zurückgeschoben, drückt die Gliederkette 6 den Schutzdeckel 5 nach vorne. Die Stützrollen 9 und 10 und damit auch der Schutzdeckel 5 werden dabei entlang der Führungsnut-Abschnitte 12 und 13 nach unten geführt, bis der Schutzdeckel 5 wieder auf der Schublade aufliegt. Auch hier wird der Bewegungsweg durch einen Endanschlag 15 am Ende des abgewinkelten Führungsnut-Abschnittes 12 begrenzt.

In den Figuren 4 bis 6 ist ein Schnellverschluss für die Abdeckung der Instrumentenablagefläche einer nicht dargestellten Behandlungseinheit gezeigt. Dieser entspricht weitgehend dem oben beschriebenen Schnellverschluss für die Schublade. Gleiche Teile werden nachfolgend daher mit gleichen Bezugszeichen versehen.

Die als Instrumentenablage dienende herausziehbare Ablageplatte 16 weist eine Abdeckung 17 auf, die aus einem hinteren Schutzdeckel 18 und einem vorderen Schutzdeckel 19 gebildet ist. Die Ablageplatte 16 ist mit dem vorderen Schutzdeckel 19 über eine elastische Gliederkette 6 verbunden. Die Gliederkette 6 ist mit ihrem einen Ende 20 am hinteren Ende 21 der Ablageplatte 16 und mit ihrem anderen Ende 22 am hinteren Ende 23 des vorderen Schutzdeckels 19 befestigt. Die Kettenaufnahme ist Bestandteil der an dem vorderen Schutzdeckel 19 angebrachten hinteren Stützrolle 24, die zusammen mit der im vorderen Bereich des vorderen Schutzdeckels 19 angeordneten Stützrolle 25 entlang der Führungsnuten 26 und 27 die Bewegung des vorderen Schutzdeckels 19 bestimmen. Die Führungsnut 26 ist in den Seitenwänden der Ausnehmung 2 in der Behandlungseinheit und die Führungsnut 27 ist in den Seitenwandabschnitten des hinteren Schutzdeckels 18 ausgebildet.

Die Führungsnut 26 weist einen abgewinkelten Abschnitt 28 auf. Bei einer Bewegung der Stützrollen 24 und 25 entlang des abgewinkelten Abschnitts 28 wird der vordere Schutzdeckel 19 abgesenkt und unter den hinteren Schutzdeckel 18 gezogen.

Der vordere Schutzdeckel 19 weist eine seitlich angeordnete Kurvenscheibe 29 auf, die mit einer an der Führungsplatte 4 angeordneten Andrückrolle 30 in Eingriff tritt. Damit wird der vordere Schutzdeckel 19 in der Endphase seiner Schließbewegung durch die Bewegung der Andrückrolle 30 entlang der Kurvenscheibe 29 zwangsweise nach oben in seine Schließstellung gedrückt.

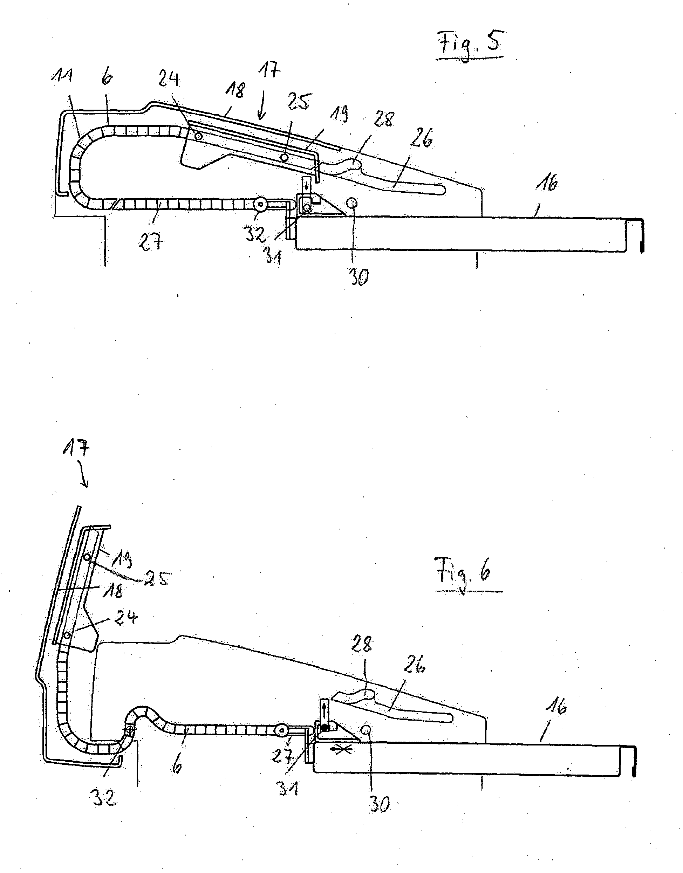

Die Funktionsweise dieser die Ablageplatte 16 der Instrumentenablage abdeckenden Verschlusseinrichtung ergibt sich aus den Fig. 4 bis 6. In Fig. 4 ist die Ablageplatte 16 in die Ausnehmung 2 eingeschoben und der vordere Schutzdeckel 19 liegt auf der Abdeckplatte 16 auf. Wird nun, wie in Fig. 5 zu ersehen ist, die Ablageplatte 16 aus der Behandlungseinheit nach vorne gezogen, wird der vordere Schutzdeckel 19 über die Stützrollen 24 und 25 entlang des abgewinkelten Abschnittes 28 der Führungsnut 26 abgesenkt und unter den hinteren Schutzdeckel 18 gezogen. Danach wird der vordere Schutzdeckel 19 entlang der Führungsnut 27 bis zu einem Anschlag unter den hinteren Schutzdeckel 18 gezogen. In dieser Endstellung lässt sich der hintere Schutzdeckel 18 mit dem darunter positionierten vorderen Schutzdeckel 19 nach oben klappen. Dadurch wird die Reinigung der Instrumentenablagefläche der Behandlungseinheit wesentlich erleichtert.

Um zu verhindern, dass in dieser Lage die Instrumentenablageplatte 16 in die Behandlungseinheit eingeschoben wird, ist ein Riegelmechanismus 31 vorgesehen, der das Einschieben der schubladenartigen Ablageplatte 16 bei geöffneter Abdeckung 17 blockiert. Damit wird eine Beschädigung der Gliederkette 6 verhindert. Diese kann sich nämlich bei hochgeklapptem vorderem Schutzdeckel 19 nicht in den vorderen Bereich der Führungsnut 26 bewegen, so dass die Gliederkette 6 beschädigt werden könnte. Ein Einschieben der Ablageplatte in die Behandlungseinheit ist nur dann möglich, wenn der hintere Schutzdeckel 18 abgesenkt ist und der vordere Schutzdeckel 19 sich unter dem hinteren Schutzdeckel befindet.

Wie im Fall der eingangs beschriebenen Schutzdeckel-Verschlusseinrichtung ist auch bei der Instrumentenablageplatte 16 die elastische Gliederkette 6 als halbsteife Kette ausgebildet, die nur in einer Richtung verformbar ist. Zur Entlastung der halbsteifen Kette im Bereich der Umlenkung während des Hochklappens der Abdeckung 17 ist in der Gliederkette 6 eine Spiralfeder eingesetzt, die als Schwenkachse 32 dient. Der vordere Schutzdeckel 19 besteht wie der Schutzdeckel 5 aus einem transparenten Kunststoff.

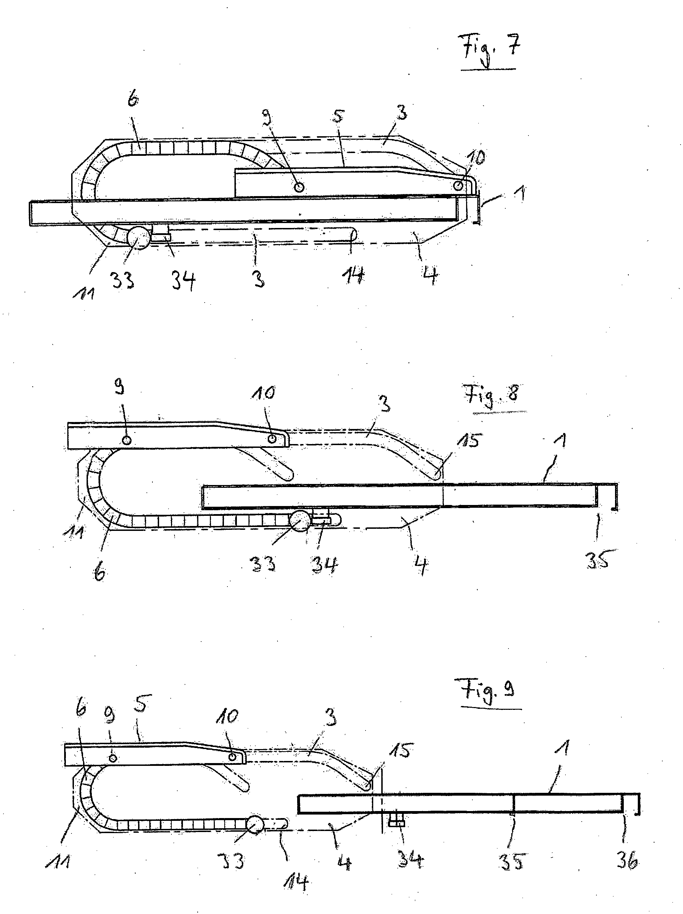

Die feste Verbindung der Gliederkette 6 mit der Schublade 1 erlaubt nur ein begrenztes Herausziehen der Schublade 1 bis zu einem ersten Schubladenanschlag 35 (Fig. 8). Die Ausziehlänge der oben beschriebenen Schublade 1 wird dabei begrenzt durch die Länge der Führungsnut 3 bis zum Endanschlag 14.

In manchen Fällen möchte der Anwender die gesamte Schubladenfläche überblicken können. Dazu sollte sich die Schublade so weit wie möglich herausziehen lassen. Um ein Ausziehen der Schublade 1 über den ersten Schubladenanschlag 35 hinaus zu ermöglichen, ist gemäß einer weiteren Ausführungsform an dem der Schublade 1 und/oder der Instrumentenablageplatte 16 zugeordneten Ende der Gliederkette eine Mitnahmestange 33 angeordnet, die mit einem Magneten 34 eine lösbare Verbindung zwischen der Schublade 1 und der Gliederkette 6 bildet. Anstatt einer Magnetverbindung kann auch eine Schnappverbindung vorgesehen sein. Bei einer lösbaren Verbindung lässt sich die Schublade bis zu einem zweiten Schubladenanschlag 36 herausziehen (Fig. 9).

Die Haltekraft der lösbaren Verbindung muss dabei so beschaffen sein, dass sie sich bei einem (normalen) Herausziehen der Schublade solange öffnet, bis die Mitnahmestange 33 den Endanschlag 14 erreicht hat. Hat die Mitnahmestange 33 den Endanschlag 14 erreicht, sollte die zusätzlich aufzuwendende Kraft, um die Verbindung zu lösen, nicht zu groß und störend sein.

### Bezugszeichenliste:

- 1: Schublade
- 2: Ausnehmung / Aufnahme für 1 und/oder 16
- 3: Führungsnut
- 4: Führungsplatte
- 5: Schutzdeckel
- 6: Gliederkette
- 7: Schubladenseitiges Ende von 6
- 8: Schutzdeckelseitiges Ende von 6
- 9: hintere Stützrolle
- 10: vordere Stützrolle
- 11: Umlenkung
- 12: Abwinklung von 3 / Führungsnut-Abschnitt
- 13: Führungsnut-Abschnitt
- 14: Endanschlag
- 15: Endanschlag
- 16: Instrumentenablageplatte
- 17: Abdeckung
- 18: hinterer Schutzdeckel
- 19: vorderer Schutzdeckel
- 20: Ablagenplattenseitiges Ende von 6
- 21: hinteres Ende von 16
- 22: Schutzdeckelseitiges Ende von 6
- 23: hinteres Ende von 19
- 24: hintere Stützrolle
- 25: vordere Stützrolle
- 26: Führungsnut in 2
- 27: Führungsnut in 18
- 28: abgewinkelter Abschnitt der Führungsnut 26
- 29: Kurvenscheibe
- 30: Andrückrolle
- 31: Riegelmechanismus
- 32: Schwenkachse
- 33: Mitnahmestange
- 34: Magnet
- 35: erster Schubladenanschlag bei fester Verbindung
- 36: zweiter Schubladenanschlag bei gelöster Verbindung

## Patentansprüche

1. Behandlungseinheit, insbesondere Instrumentenschrank für medizinische Arbeitsplätze mit mindestens einer herausziehbaren Schublade (1) und/oder einer Instrumentenablageplatte (16), die in Ausnehmungen (2) in der Behandlungseinheit auf seitlichen Schienen gelagert sind, wobei die Schublade (1) und/oder die Instrumentenablageplatte (16) der Behandlungseinheit mit einem Schutzdeckel (5; 18, 19)) bewegbar verbunden ist, **dadurch gekennzeichnet, dass** die Schublade (1) und/oder die Instrumentenablageplatte (16) der Behandlungseinheit mit dem Schutzdeckel (5; 18, 19) über wenigstens eine elastische Gliederkette (6) bewegbar verbunden ist, deren eines Ende (7; 20) am hinteren Ende der Schublade (1) und/oder der Instrumentenablageplatte (16) und deren anderes Ende (8; 22) am hinteren Ende des Schutzdeckels (5; 19) befestigt ist, dass an den Seitenwänden der Ausnehmung (2) in der Behandlungseinheit Führungsplatten (4) angeordnet sind, in denen wenigstens eine annähernd U-förmige Führungsnut (3; 27) ausgebildet ist, in der die Gliederkette (6) geführt ist, dass an den Seiten des Schutzdeckels (5; 19) Stützrollen (9, 10; 24, 25) angeordnet sind, die in die Führungsnut(en) (3; 26) eingreifen, dass die Führungsnut (3; 26) abgewinkelte Abschnitte (12, 13; 28) aufweist und dass bei einer Bewegung der Stützrollen (9, 10; 24, 25) entlang der abgewinkelten Abschnitte (12, 13; 28) der Führungsnut (3; 26) der Schutzdeckel (5; 19) je nach Bewegungsrichtung der Gliederkette (6) angehoben oder abgesenkt wird.

2. Behandlungseinheit für medizinische Arbeitsplätze mit mindestens einer herausziehbaren Schublade (1), die in Ausnehmungen (2) in dem Instrumentenschrank auf seitlichen Schienen gelagert ist, nach Anspruch 1, **dadurch gekennzeichnet, dass** zwei parallel zueinander verlaufende abgewinkelte Führungsnut-Abschnitte (12, 13) in der seitlichen Führungsplatte ausgebildet sind, in die die an dem Schutzdeckel (5) angeordneten Stützrollen (9, 10) eingreifen.

3. Behandlungseinheit für medizinische Arbeitsplätze mit einer als Instrumentenablage dienenden herausziehbaren Ablageplatte (16), die mit einem Schutzdeckel abdeckbar ist und die in einer Ausnehmung (2) in dem Instrumentenschrank auf seitlichen Schienen gelagert ist, nach Anspruch 1, **dadurch gekennzeichnet, dass** die Ablageplatte (16) einen aufschwenkbaren hinteren Schutzdeckel (18) aufweist, der mit einem vorderen Schutzdeckel (19) die Abdeckung (17) der Ablageplatte (16) bildet, dass die Ablageplatte (16) mit dem vorderen Schutzdeckel (19) über wenigstens eine elastische Gliederkette (6) bewegbar verbunden ist, deren eines Ende (20) am hinteren Ende (21) der Ablageplatte (16) und deren anderes Ende (22) am hinteren Ende (23) des vorderen Schutzdeckels (19) befestigt ist, dass an den Seiten des vorderen Schutzdeckels (19) Stützrollen (24, 25) angeordnet sind, die in Führungsnuten (26) eingreifen, die in den Seitenwänden der Ausnehmung (2) in der Behandlungseinheit und in Seitenwandabschnitten des hinteren Schutzdeckels (19) ausgebildet sind, dass die Führungsnut (26) abgewinkelte Abschnitte (28) aufweist und dass bei einer Bewegung der Stützrollen (24, 25) entlang des wenigstens einen abgewinkelten Abschnitts (28) der Führungsnut (26) der vordere Schutzdeckel (19) abgesenkt und unter den hinteren Schutzdeckel (18) gezogen wird.

4. Behandlungseinheit nach Anspruch 3, **dadurch gekennzeichnet, dass** der vordere Schutzdeckel (19) seitlich angeordnete Kurvenscheiben (29) aufweist, die mit an der Führungsplatte (4) angeordneten Andrückrollen (30) in Eingriff treten.

5. Behandlungseinheit nach Anspruch 3 oder 4, **dadurch gekennzeichnet, dass** der hintere Schutzdeckel (18) bei zurückgezogenem vorderen Schutzdeckel (19) mit dem darunter positionierten vorderen Schutzdeckel (19) aufschwenkbar ist.

6. Behandlungseinheit nach einem der vorhergehenden Ansprüche, **dadurch gekennzeichnet, dass** die hinteren Stützrollen (9; 24) an den Seiten des mit der Gliederkette (6) verbundenen verschiebbaren Schutzdeckels (5; 19) eine Kettenaufnahme aufweisen.

7. Behandlungseinheit nach einem der Ansprüche 3 bis 6, **dadurch gekennzeichnet, dass** die Behandlungseinheit einen Riegelmechanismus (31) aufweist, der das Einschieben der Instrumentenablageplatte (16) bei geöffneter Abdeckung (17) blockiert.

8. Behandlungseinheit nach einem der vorhergehenden Ansprüche, **dadurch gekennzeichnet, dass** die elastische Gliederkette (6) eine halbsteife Kette ist, die nur in einer Richtung verformbar ist.

9. Behandlungseinheit nach einem der vorhergehenden Ansprüche, **dadurch gekennzeichnet, dass** in den Führungsnuten (3; 26) der gegenüberliegenden Führungsplatten (4) jeweils eine elastische Gliederkette (6) eingreift.

10. Behandlungseinheit nach einem der vorhergehenden Ansprüche, **dadurch gekennzeichnet, dass** die Schutzdeckel (5; 19) aus einem transparenten Kunststoff bestehen.

11. Behandlungseinheit nach einem der vorhergehenden Ansprüche, **dadurch gekennzeichnet, dass** die Verbindung zwischen der Schublade (1) und/oder der Instrumentenablageplatte (16) mit der Gliederkette (6) lösbar ist.

12. Behandlungseinheit nach Anspruch 11, **dadurch gekennzeichnet, dass** an dem der Schublade (1) und/oder der Instrumentenablageplatte (16) zugewandten Ende (7; 20) der Gliederkette (6) eine Mitnahmestange (33) angeordnet ist, die mit einem an der Schublade (1) bzw. der Instrumentenablageplatte (16) angebrachten Magnet (34) eine lösbare Verbindung bildet.

13. Behandlungseinheit nach Anspruch 11, **dadurch gekennzeichnet, dass** zwischen dem der Schublade (1) und/oder der Instrumentenablageplatte (16) zugewandten Ende (7; 20) der Gliederkette (6) eine Schnappverbindung vorgesehen ist.

14. Behandlungseinheit nach Anspruch 12 oder 13, **dadurch gekennzeichnet, dass** die Haltekraft des Magneten (34) bzw. der Schnappverbindung zwischen Schublade (1) bzw. Instrumentenablageplatte (16) und Gliederkette (6) so groß ist, dass die Verbindung bei einem normalen Herausziehen der Schublade (1) bzw. der Instrumentenablageplatte (16) erst dann öffnet, wenn die Mitnahmestange (33) den Endanschlag (14) in der Führungsnut (3; 27) erreicht.

## Claims

1. A treatment unit, in particular an instrument cabinet for medical workstations with at least one pull-out drawer (1) and/or an instrument storage plate (16), which are mounted in recesses (2) on lateral rails in the treatment unit, wherein the drawer (1) and/or the instrument storage plate (16) of the treatment unit is movably connected with a protective cover (5; 18, 19), **characterised in that** the drawer (1) and/or the instrument storage plate (16) of the treatment unit is movably connected with the protective cover (5; 18 19) via at least one elastic link chain (6), one end (7; 20) of which is fastened at the rear end of the drawer (1) and/or the instrument storage plate (16) and the other end (8; 22) of which is fastened at the rear end of the protective cover (5; 19), **in that** the side walls of the recess (2) in the treatment unit have guiding plates (4) arranged in them, in which at least one approximately U-shaped guiding groove (3; 27) is formed, in which the link chain (6) is guided, **in that** the sides of the protective cover (5; 19) have support rollers (9, 10; 24, 25) arranged in them, which engage in the guiding grooves (3; 26), **in that** the guiding groove (3; 26) comprises angled sections (12, 13; 28) and **in that** when the support rollers (9, 10; 24, 25) are moved along the angled sections (12, 13; 28) of the guiding groove (3; 26), the protective cover (5; 19) is raised or lowered), depending on the direction of movement of the link chain (6).

2. The treatment unit for medical workstations with at least one pull-out drawer (1) mounted on lateral rails in recesses (2) in the instrument cabinet, according to claim 1, **characterised in that** two angled guiding groove sections (12, 13) extending in parallel are formed in the lateral guiding plate, into which the support rollers (9, 10) arranged on the protective cover (5) engage.

3. A treatment unit for medical workstations with a pull-out storage plate (16) serving as a storage shelf for depositing instruments, which can be covered by a protective cover and which is mounted on lateral rails in a recess (2) in the instrument cabinet, according to claim 1, **characterised in that** the storage plate (16) comprises a swing-open rear protective cover (18) which together with a front protective cover (19) forms the cover (17) of the storage plate (16), **in that** the storage plate (16) is movably connected with the front protective cover (19) via at least one elastic link chain (6), one end (20) of which is attached at the rear end (21) of the storage plate (16) and the other end (22) of which is attached at the rear end (23) of the front protective cover (19), **in that** support rollers (24, 25) are arranged at the sides of the front protective cover (19) which engage in guiding grooves (26) which are formed in the side walls of the recess (2) in the treatment unit and in the side wall sections of the rear protective cover (18), **in that** the guiding groove (26) comprises angled sections (28) and **in that** when the support rollers (24, 25) are moved along at least one angled section (28) of the guiding groove (26), the front protective cover (19) is lowered and is drawn under the rear protective cover (18).

4. The treatment unit according to claim 3, **characterised in that** the front protective cover (19) comprises laterally arranged cam discs (29) which enter into engagement with pressure rollers (30) arranged in the guide plate (4).

5. The treatment unit according to claim 3 or 4, **characterised in that** the rear protective cover (18) can be swung open together with front protective cover (19) arranged underneath it when the front protective cover (19) is retracted.

6. The treatment unit according to one of the preceding claims, **characterised in that** the rear support rollers (9; 24) comprise a chain holder on the sides of the movable protective cover (5; 19) connected with the link chain (6).

7. The treatment unit according to one of claims 3 to 6, **characterised in that** the treatment unit comprises a latch mechanism (31) which blocks insertion of the instrument storage plate (16) when the cover (17) is open.

8. The treatment unit according to one of the preceding claims, **characterised in that** the elastic link chain (6) is a semi-rigid chain which is deformable in one direction only.

9. The treatment unit according to one of the preceding claims, **characterised in that** an elastic link chain (6) respectively engages into the guiding grooves (3; 26) of the opposite guide plates (4).

10. The treatment unit according to one of the preceding claims, **characterised in that** the protective covers (5; 19) consist of a transparent plastic.

11. The treatment unit according to one of the preceding claims, **characterised in that** the connection between the drawer (1) and/or the instrument storage plate (16) can be undone with the link chain (6).

12. The treatment unit according to one of the preceding claims, **characterised in that** a drive rod (33) is arranged at the end (7; 20) of the link chain (6) facing the drawer (1) and/or the instrument storage plate (16), which forms a detachable connection with a magnet (34) provided at the drawer (1) or the instrument storage plate (16).

13. The treatment unit according to claim 1, **characterised in that** a snap-on connection is provided between the end (7; 20) of the link chain (6) facing the drawer (1) and/or facing the instrument storage plate (16).

14. The treatment unit according to claim 12 or 13, **characterised in that** the retaining force of the magnet (34) or the snap-on connection between drawer (1) or instrument storage plate (16) and link chain (6) is so strong that when the drawer (1) or the instrument storage plate (16) is pulled out as normal, the connection does not open until the drive rod (33) has reached the final stop (14) in the guiding groove (3; 27).

## Revendications

1. Unité de traitement, en particulier armoire à instruments pour postes de travail médicaux avec au moins un tiroir (1) ouvrable et/ou un plateau de rangement d'instruments (16), lesquels sont montés dans des évidements (2) dans l'unité de traitement sur des rails latéraux, le tiroir (1) et/ou le plateau de rangement d'instruments (16) de l'unité de traitement étant relié(s) de manière mobile avec un couvercle de protection (5 ; 18, 19), **caractérisée en ce que** le tiroir(1) et/ou le plateau de rangement d'instruments (16) de l'unité de traitement est/sont relié(s) de manière mobile au couvercle de protection (5 ; 18, 19) via au moins une chaîne à maillons (6) élastique dont l'une des extrémités (7 ; 20) est fixée à l'extrémité arrière du tiroir (1) et/ou du plateau de rangement d'instruments (16) et dont l'autre des extrémités (8 ; 22) est fixée à l'extrémité arrière du couvercle de protection (5 ; 19), **en ce que** des plaques de guidage (4) sont disposées sur les parois latérales de l'évidement (2) dans l'unité de traitement, dans lesquelles plaques est formée au moins une rainure de guidage (3 ; 27) sensiblement en forme de U dans laquelle la chaîne à maillons (6) est guidée, **en ce que** des rouleaux de support (9, 10 ; 24, 25) sont disposés sur les côtés du couvercle de support (5 ; 19) lesquels se mettent en prise dans la/les rainure(s) de guidage (3 ; 26), **en ce que** la rainure de guidage (3 ; 26) présente des parties coudées (12, 13 ; 28) et **en ce qu'**en cas d'un déplacement des rouleaux de support (9, 10 ; 24, 25) le long des parties coudées (12, 13 ; 28) de la rainure de guidage (3 ; 26), le couvercle de protection (5 ; 19) est soulevé ou abaissé en fonction du sens de déplacement de la chaîne à maillons (6).

2. Unité de traitement pour postes de travail médicaux avec au moins un tiroir (1) ouvrable selon la revendication 1 qui est monté dans des évidements (2) dans l'armoire à instruments sur des rails latéraux, **caractérisée en ce que** deux parties de rainure de guidage coudées (12, 13) s'étendant parallèlement l'une à l'autre sont formées dans la plaque de guidage latérale, dans lesquelles parties de rainure les rouleaux de support (9, 10) disposés sur le couvercle de protection (5) sont en prise.

3. Unité de traitement pour postes de travail médicaux avec un plateau de rangement (16) ouvrable selon la revendication 1 servant de rangement d'instruments, lequel peut être recouvert par un couvercle de protection et lequel est monté dans un évidement (2) dans l'armoire à instruments sur des rails latéraux, **caractérisée en ce que** le plateau de rangement (16) présente un couvercle de protection arrière (18) ouvrable par pivotement lequel forme, avec un couvercle de protection avant (19), le recouvrement (17) du plateau de rangement (16), **en ce que** le plateau de rangement (16) est relié de manière mobile au couvercle de protection avant (19) via au moins une chaîne à maillons (6) élastique dont l'une des extrémités (20) est fixée à l'extrémité arrière (21) du plateau de rangement (16) et dont l'autre extrémité (22) est fixée à l'extrémité arrière (23) du couvercle de protection avant (19), **en ce que** des rouleaux de support (24, 25) sont disposés sur les côtés du couvercle de protection avant (19), lesquels sont en prise dans des rainures de guidage (26) formées dans les parois latérales de l'évidement (2) dans l'unité de traitement et dans les parties de paroi latérale du couvercle de protection arrière (18), **en ce que** la rainure de guidage (26) présente des parties coudées (28) et **en ce qu'**en cas de déplacement des rouleaux de support (24, 25) le long de cette au moins une partie coudée (28) de la rainure de guidage (26), le couvercle de protection avant (19) et abaissé et est tiré sous le couvercle de protection arrière (18).

4. Unité de traitement selon la revendication 3, **caractérisée en ce que** le couvercle de protection avant (19) présente des cames (29) disposées latéralement, lesquelles se mettent en prise avec des rouleaux presseurs (30) disposés sur la plaque de guidage (4).

5. Unité de traitement selon la revendication 3 ou 4, **caractérisée en ce que**, lorsque le couvercle de protection avant (19) est retiré, le couvercle de protection arrière (18) peut être ouvert en pivotant avec le couvercle de protection avant (19) positionné en-dessous.

6. Unité de traitement selon l'une des revendications précédentes, **caractérisée en ce que** les rouleaux de support arrières (9 ; 24) présentent un logement de chaîne sur les côtés du couvercle de protection (5 ; 19) coulissant relié à la chaîne à maillons (6).

7. Unité de traitement selon l'une des revendications 3 à 6, **caractérisée en ce que** l'unité de traitement présente un mécanisme de verrouillage (31) qui bloque l'insertion du plateau de rangement d'instruments (16) en cas de recouvrement (17) ouvert.

8. Unité de traitement selon l'une des revendications précédentes, **caractérisée en ce que** la chaîne à maillons élastique (6) est une chaîne semi-rigide pouvant se déformer dans une seule direction seulement.

9. Unité de traitement selon l'une des revendications précédentes, **caractérisée en ce que**, respectivement une chaîne à maillons élastique (6) est en prise dans les rainures de guidage (3 ; 26) des plaques de guidage (4) situées en vis-à-vis.

10. Unité de traitement selon l'une des revendications précédentes, **caractérisée en ce que** les couvercles de protection (5 ; 19) se composent d'un plastique transparent.

11. Unité de traitement selon l'une des revendications précédentes, **caractérisée en ce que** la liaison entre le tiroir (1) et/ou le plateau de rangement d'instruments (16) et la chaîne à maillons (6) est détachable.

12. Unité de traitement selon la revendication 11, **caractérisée en ce qu'**une tige d'entraînement (33) est disposée à l'extrémité (7 ; 20) de la chaîne à maillons (6) tournée vers le tiroir (1) et/ou le plateau de rangement d'instruments (16), laquelle tige forme une liaison détachable avec un aimant (34) mis en place sur le tiroir (1) et/ou le plateau de rangement d'instruments (16).

13. Unité de traitement selon la revendication 11, **caractérisée en ce que** l'on prévoit une liaison par encliquetage entre l'extrémité (7 ; 20) de la chaîne à maillons (6) tournée vers le tiroir (1) et/ou le plateau de rangement d'instruments (16).

14. Unité de traitement selon la revendication 12 ou 13, **caractérisée en ce que** la force de maintien de l'aimant (34) ou de la liaison par encliquetage entre le tiroir (1) ou le plateau de rangement d'instruments (16) et la chaîne à maillons (6) est si forte que, lorsque l'on ouvre le tiroir (1) ou le plateau de rangement d'instruments (16) de manière normale, la liaison s'ouvre seulement si la tige d'entraînement (33) atteint la butée finale (14) dans la rainure de guidage (3 ; 27).
